(19) Europäisches Patentamt
European Patent Office
Office européen des brevets

(11) Veröffentlichungsnummer: **0 098 794**
**B1**

(12) # EUROPÄISCHE PATENTSCHRIFT

(45) Veröffentlichungstag der Patentschrift: **27.04.88**

(21) Anmeldenummer: **83730066.4**

(22) Anmeldetag: **01.07.83**

(51) Int. Cl.⁴: **C 07 C 177/00**, C 07 C 59/62, C 07 C 69/734, C 07 C 103/737, A 61 K 31/557 // C07D317/72

(54) **Neue Carbacycline, Verfahren zu ihrer Herstellung und ihre Verwendung als Arzneimittel.**

(30) Priorität: **02.07.82 DE 3225288**

(43) Veröffentlichungstag der Anmeldung: **18.01.84 Patentblatt 84/3**

(45) Bekanntmachung des Hinweises auf die Patenterteilung: **27.04.88 Patentblatt 88/17**

(84) Benannte Vertragsstaaten: **AT BE CH DE FR GB IT LI LU NL SE**

(56) Entgegenhaltungen: **EP-A-0 055 208**
**GB-A-2 070 596**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Skuballa, Werner, Dr., Olwenstrasse 25, D-1000 Berlin 28 (DE)**
Erfinder: **Radüchel, Bernd, Dr., Gollanczstrasse 132, D-1000 Berlin 28 (DE)**
Erfinder: **Vorbrüggen, Helmut, Prof. Dr., Wilkestrasse 7, D-1000 Berlin 27 (DE)**
Erfinder: **Casals- Stenzel, Jorge, Dr., Sertoriusring 295, D-6500 Mainz 21 (Finthen) (DE)**
Erfinder: **Mannesmann, Gerda, Dr., Sachsenring 22/24, D-5000 Köln 1 (DE)**
Erfinder: **Town, Michael Harold, Dr., Buchsbaumweg 3, D-1000 Berlin 47 (DE)**

EP 0 098 794 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft neue Prostacyclin-Derivate, Verfahren zu ihrer Herstellung sowie ihre Verwendung als Arzneimittel.

In den deutschen Offenlegungsschriften DE-OS-2 845 770, 2 900 352, 2 902 442, 2 904 655, 2 909 088, 3 048 906 und 2 912 409 werden (5E)- und (5Z)-6a-Carbaprostaglandin-$I_2$-Analoga beschrieben, und aus der britischen Patentanmeldung 2 070 596 sind 5-Fluor-Carbacycline bekannt. Die Nomenklatur der erfindungsgemäßen Verbindungen basiert auf einem Vorschlag von Morton und Brokow (J.Org.Chem. 44, 2880 [1979]. Bei der Synthese dieser Verbindungn, entstehen stets zwei Doppelbindungsisomere, die durch den Zusatz (5E) oder (5Z) charakterisiert werden. Die beiden Isomeren dieses Prototyps werden durch folgende Strukturformel verdeutlicht:

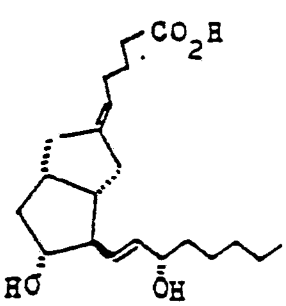

(5E)-6a-Carbaprostaglandin-$I_2$

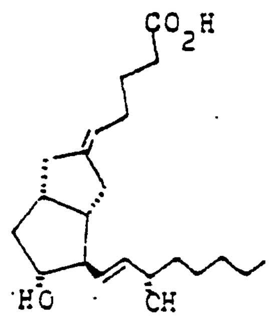

(5Z)-6a-Carbaprostaglandin-$I_2$

Aus dem sehr umfangreichen Stand der Technik der Prostacycline und ihrer Analoga weiß man, daß diese Stoffklasse aufgrund ihrer biologischen und pharmakologischen Eigenschaften zur Behandlung von Säugetieren, einschließlich Menschen geeignet ist. Ihre Verwendung als Arzneimittel stößt jedoch häufig auf Schwierigkeiten, da sie eine für therapeutische Zwecke zu kurze Wirkungsdauer besitzen. Alle Strukturveränderungen haben das Ziel, die Wirkungsdauer sowie die Selektivität der Wirksamkeit zu steigern.

Es wurde nun gefunden, daß durch Ersatz der Methylengruppe in der 3-Position durch Sauerstoff und des Wasserstoffatoms in der 5-Position durch ein Fluoratom eine längere Wirkungsdauer, eine größere Selektivität und eine bessere Wirksamkeit erzielt werden können. Die erfindungsgemäßen Verbindungen wirken blutdrucksenkend und bronchodilatatorisch. Sie sind außerdem zur Vasodilatation, Inhibierung der Thrombocytenaggregation und der Magensäuresekretion geeignet.

Die Erfindung betrifft Carbacyclinderivate der allgemeinen Formel I

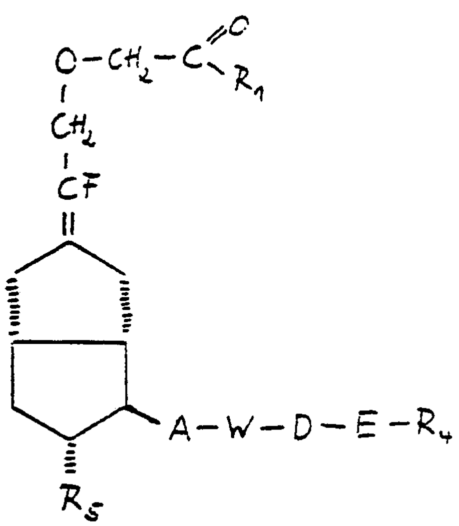

(I),

$R_1$ den Rest $OR_2$, wobei $R_2$ Wasserstoff, Alkyl mit 1-4 C-Atomen, Cycloalkyl mit 5-6 C-Atomen, Phenyl, 1-Naphthyl, 2-Naphthyl, den Rest

$$-CH_2-\overset{\overset{\displaystyle O}{\|}}{C}-Aryl,$$

wobei Aryl einen durch Phenyl, $C_1$-$C_2$-Alkoxy, Chlor oder Brom subatituierte, Phenyl-, 1-Naphthyl- oder 2-Napthylrest darstellt, oder eine 5- oder 6-gliedrige heterocyclische Gruppe mit einem N-, O- oder S-Atom bedeuten kann, oder $R_1$ den Rest $NHR_3$ mit $R_3$ in der Bedeutung eines organischen Carbon- oder Sulfonsäurerestes mit 1-15 C-Atome, oder des Restes $R_2$,

A eine $-CH_2-CH_2-$, trans-CH=CH- oder -C≡C-Gruppe,

W eine freie oder funktionell abgewandelte Hydroxymethylengruppe oder eine freie oder funktionell abgewandelte

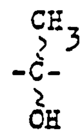

Gruppe, wobei die OH-Gruppe α- oder β-ständig sein kann,

D die Gruppe

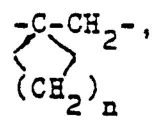

eine geradkettige gesättigte Alkylengruppe mit 1-5 C-Atomen, eine verzweigte gesättigte oder eine geradkettige oder verzweigte ungesättigte Alkylengruppe mit 2-5 C-Atomen, die gegebenenfalls durch Fluoratome substituiert sein können,

n die Zahl 1, 2 oder 3 bedeuten kann,

E eine Direktbindung, eine -C≡C-Gruppe oder eine $-CR_6=CR_7$-Gruppe darstellt, wobei $R_6$ Wasserstoff oder $C_1$-$C_4$-Alkyl und $R_7$ Wasserstoff, $C_1$-$C_4$-Alkyl Chlor oder Brom bedeuten,

$R_4$ eine Alkylgruppe mit 1-10 C-Atomen, eine Cycloalkylgruppe mit 3-6 C-Atomen, Phenyl oder eine 5- oder 6-gliedrige heterocycliache Gruppe mit einem N-, O- oder S-Atom,

$R_5$ eine freie oder funktionell abgewandelte Hydroxygruppe, und

falls $R_2$ die Bedeutung eines Wasserstoffatoms hat, deren Salze mit physiologisch verträglichen Basen bedeuten.

Die Verbindungen der Formel I stellen sowohl (5E)- als auch (5Z)-Isomere dar.

Als Alkylgruppen $R_2$ sind gerad- oder verzweigtkettige Alkylgruppen mit 1-10 C-Atomen zu betrachten, wie beispielsweise Methyl, Äthyl, Propyl, Isopropyl, Butyl, Isobutyl, tert.-Butyl, Pentyl, Isopentyl, Neopentyl, Heptyl, Hexyl, Decyl.

Als bevorzugte Alkylgruppen $R_2$ mit 1-4 C-Atomen sind z. B. Methyl, Äthyl, Propyl, Isobutyl und Butyl zu nennen.

Arylgruppen $R_2$ sind Phenyl-1-Naphthyl und 2-Naphthyl.

Die Cycloalkylgruppe $R_2$ kann im Ring 5 und 6 Kohlenstoffatome enthalten. Beispielsweise seien genannt Cyclopentyl-, Cyclohexyl.

Heterocyclische Gruppen $R_2$ sind 5- und 6-gliedrige Heterocyclen, die 1 Heteroatom, das Stickstoff, Sauerstoff oder Schwefel ist, enthalten. Beispielweise seien genannt 2-Furyl, 3-Furyl, 2-Thienyl, 3-Thienyl, 2-Pyridyl, 3-Pyridyl, 4-Pyridyl u. a.

Der Aryl-Rest in der $-CH_2-\underset{\underset{\displaystyle O}{\|}}{C}-Aryl$-Gruppe

von $R_2$ stellt Phenyl, α- oder β-Naphthyl das, die durch Phenyl, $C_1$-$C_2$-Alkoxy, Chlor oder Brom substituiert sein können.

Als Säurerest $R_3$ kommen physiologisch verträgliche Säurereste in Frage. Bevorzugte Säuren sind organische Carbonsäuren und Sulfonsäuren mit 1-15 Kohlenstoffatomen, die der aliphatischen, cycloaliphatischen, aromatischen, aromatisch-aliphatischen und heterocyclischen Reihe angehören. Diese Säuren können gesättigt, ungesättigt und/oder mehrbasisch und/oder in üblicher Weise substituiert sein. Als Beispiele für die Substituenten seien $C_1$-$C_4$-Alkyl-, Hydroxy-, $C_1$-$C_4$-Alkoxy-, Oxo- oder Aminogruppen oder Halogenatome (F, Cl, Br) erwähnt.

Beispielweise seien folgende Carbonsäuren genannt: Ameisensäure, Essigsäure, Propionsäure, Buttersäure, Isobuttersäure, Valeriansäure, Isovaleriansäure, Capronsäure, Önanthsäure, Caprylsäure, Pelargonsäure, Caprinsäure, Undecylsäure, Laurinsäure, Tridecylsäure, Myristinsäure, Pentadecylsäure, Trimethylessigsäure, Diäthylessigsäure, tert.-Butylessigsäure, Cyckopropylessigsäure, Cyclopentylessigsäure, Cyclohexylessigsäure,

Cyclopropancarbonsäure, Cyclohexancarbonsäure, Phenylessigsäure, Phenoxyessigsäure, Methoxyessigsäure, Äthoxyessigsäure, Mono-, Di- und Trichloressigsäure, Aminoessigsäure, Diäthylaminoessigsäure, Piperidinoessigsäure, Morpholinoessigsäure, Milchsäure, Bernsteinsäure, Adipinsäure, Benzoesäure, mit Halogen-, Trifluormethyl-, Hydroxy-, Alkoxy- oder Carboxy-Gruppen substituierte Benzoesäuren, Nikotinsäure, Isonikotinsäure, Furan-2-carbonsäure, Cyclopentylpropionsäure. Als besonders bevorzugte Acylreste werden solche mit bis zu 10 Kohlenstoffatomen betrachtet. Als Sulfonsäuren kommen beispielweise Methansulfonsäure, Äthansulfonsäure, Isopropansulfonsäure, β-Chloräthansulfonsäure, Butansulfonsäure, Cyclopentansulfonsäure, Cyclohexansulfonsäure, Benzolsulfonsäure, p-Toluolsulfonsäure, p-Chlorbenzolsulfonsäure, N,N-Dimethylaminosulfonsäure, N,N-Diäthylaminosulfonsäure, N,N-Bis-(β-chloräthyl)aminosulfonsäure, N,N-Diisobutylaminosulfonsäure, N,N-Dibutylaminosulfonsäure, Pyrrolidino-, Piperidino-, Piperazino-, N-Methylpiperazino- und Morpholinosulfonsäure in Frage.

Die Hydroxygruppen $R_5$ und in W können funktionell abgewandelt sein, beispielsweise durch Verätherung oder Veresterung, wobei die freien oder abgewandelten Hydroxygruppen in W α- oder β-ständig sein können, wobei freie Hydroxygruppen bevorzugt sind.

Als Äther- und Acylreste kommen die dem Fachmann bekannten Reste in Betracht. Bevorzugt sind leicht abspaltbare Ätherreste wie beispielweise der Tetrahydropyranyl-, Tetrahydrofuranyl- α-Äthoxyäthyl-, Trimethylsilyl-, Dimethyl-tert.-butyl-silyl- und Tribenzyl-silylrest. Als Acyclreste kommen die gleichen wie für $R_3$ genannt in Frage; namentlich genannt seien beispielsweise Acetyl, Propionyl, Butyryl, Benzoyl.

Als Alkylgruppe $R_4$ kommen gerad- und verzweigtkettige, gesättigte und ungesättigte Alkylreste, vorzugsweise gesättigte, mit 1-10, insbesondere 1-7 C-Atomen, in Frage, die gegebenenfalls durch gegebenenfalls substituiertes Aryl substituiert sein können. Beispielweise genannt seien Methyl-, Äthyl-, Propyl-, Butyl-, Isobutyl-, tert.-Butyl-, Pentyl-, Hexyl-, Heptyl-, Octyl-, Butenyl-, Isobutenyl-, Propenyl-, Pentenyl-, Hexenyl-, Benzyl- und p-Chlorbenzyl.

Die Cycloalkylgruppe $R_4$ enthält im Ring 3-6 Kohlenstoffatome. Beispielsweise seien genannt Cyclopropyl, Cyclopentyl, Cyclohexyl.

Heterocyclische Gruppen $R_4$ sind 5- und 6-gliedrige Heterocyclen, die 1 Heteroatom, das Stickstoff, Sauerstoff oder Schwefel ist, enthalten. Beispielsweise seien genannt 2-Furyl, 2-Thienyl, 2-Pyridyl, 3-Pyridyl-, 4-Pyridyl, 3-Furyl, 3-Thienyl u.a.

Alkylengruppe D sind geradkettige oder verzweigtkettige, gesättigte und ungesättigte Alkylenreste mit bis zu 5 C-Atomen, vorzugsweise gesättigte mit 1-5 C-Atomen, die gegebenenfalls durch Fluoratome substituiert sein können. Beispielsweise seien genannt: Methylen, Fluormethylen, Äthylen, 1,2-Propylen, Äthyläthylen, Trimethylen, Tetramethylen, Pentamethylyn, 1-Methyl-tetramethylen, 1-Methyl-trimethylen.

Die Alkylgruppen $R_6$ und $R_7$ stellen geradkettige oder verzweigte gesättigte Alkylgruppen mit 1-4 C-Atomen dar, wie sie bereits für $R_2$ und $R_4$ genannt wurden. $R_6$ und $R_7$ als Halogen können Chlor und Brom, vorzugsweise Chlor sein.

Zur Salzbildung mit den freien Säuren ($R_2 = H$) sind anorganische und organische Basen geeignet, wie sie dem Fachmann zur Bildung physiologisch verträglicher Salze bekannt sind. Beispielsweise seien genannt: Alkalihydroxide, wie Natrium- und Kaliumhydroxid, Erdalkalihydroxide, wie Calciumhydroxid, Ammoniak, Amine, wie Äthanolamin, Diäthanolamin, Triäthanolamin, N-Methylglucamin, Morpholin, Tris-(hydroxymethyl)-methylamin usw.

Die Erfindung betrifft ferner ein Verfahren zur Herstellung der erfindungsgemäßen Carbacyclinderivate der allgemeinen Formel I, dadurch gekennzeichnet, daß man in an sich bekannter Weise eine Verbindung der allgemeinen Formel II,

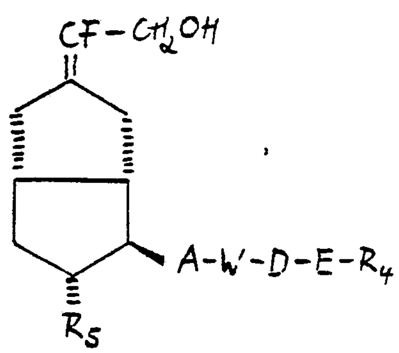

II,

worin $R_4$, $R_5$ , A, W, D und E die obenangegebenen Bedeutungen aufweisen, gegebenenfalls nach Schutz anwesender freier Hydroxygruppen mit einem Halogenessigsäurederivat der allgemeinen Formel III,

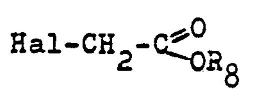

III,

wobei Hal ein Chlor- Brom- oder Jodatom und $R_8$ einen Alkylrest oder Trialkyl-silylrest mit $C_1$-$C_4$-Alkylgruppen oder ein Alkalimetall (Na, Li, K) bedeutet, in Gegenwart einer Base veräthert und gegebenenfalls anschließend

in beliebiger Reihenfolge Isomere trennt und/oder geschützte Hydroxygruppen freisetzt und/oder freie Hydroxygruppen verestert oder veräthert und/oder eine freie Carboxylgruppe verestert und/oder eine veresterte Carboxylgruppe verseift oder eine Carboxylgruppe in ein Amid oder mit einer physiologisch verträglichen Base in ein Salz überführt.

Die Umsetzung der Verbindung der allgemeinen Formel II mit einem Halogenessigsäurederivat der allgemeinen Formel III wird bei Temperaturen von 0°C bis 100°C, vorzugsweise 10°C bis 80°C, in einem aprotischen Lösungsmittel oder Lösungsmittelgemisch, beisbielsweise Dimethylsulfoxid, Dimethylformamid, Tetrahydrofuran usw., vorgenommen. Als Basen kommen die dem Fachmann für Verätherungen bekannter Basen in Frage, beispielsweise Natriumhydrid, Kalium- tert.-butylat, Butyllithium usw.

Die Verseifung der Carbacyclinester wird nach den dem Fachmann bekannten Methoden durchgeführt, wie beispielsweise mit basischen Katalysatoren.

Die Einführung der Estergruppe -$OR_2$ für $R_1$, bei welcher $R_2$ eine Alkylgruppe mit 1-10 C-Atomen darstellt, erfolgt nach den dem Fachmann bekannten Methoden. Die Carboxyverbindungen werden beispielsweise mit Diazokohlenwasserstoffen in an sich bekannter Weise umgesetzt. Die Veresterung mit Diazokohlenwasserstoffen erfolgt zum Beispiel dadurch, daß man eine Lösung des Diazokohlenwasserstoffes in einem inerten Lösungsmittel, vorzugsweise in Diäthyläther mit der Carboxyverbindung in dem gleichen oder in einem anderen inerten Lösungsmittel, wie zum Beispiel Methylenchlorid, vermischt. Nach beendeter Umsetzung in 1 bis 30 Minuten wird das Lösungsmittel entfernt und der Ester in üblicher Weise gereinigt. Diazoalkane sind entweder bekannt oder können nach bekannten Methoden hergestellt werden [Org.Reactions Bd. 8, Seiten 389-394 (1954)].

Die Einführung der Estergruppe -$OR_2$ für $R_1$, bei welcher $R_2$ eine substituierte oder unsubstituierte Arylgruppe darstellt, erfolgt nach den dem Fachmann bekannten Methoden. Beispielsweise werden die Carboxyverbindungen und die entsprechenden Arylhydroxyverbindungen mit Dicyclohexylcarbodiimid in Gegenwart einer geeigneten Base, beispielsweise Pyridin oder Triäthylamin, in einem inerten Lösungsmittel umgesetzt. Als Lösungsmittel kommen Methylenchlorid, Äthylenchlorid, Chloroform, Essigester, Tetrahydrofuran, vorzugsweise Chloroform, in Frage. Die Reaktion wird bei Temperaturen zwischen -30°C und +50°C, vorzugsweise bei +10°C, durchgeführt.

Die Einführung der Estergruppe -OR für R kann auch durch Umsetzung des Carboxylatanions mit dem entsprechenden Alkylhalogenid oder -Halogenketon

$$(Hal-CH_2-\overset{O}{\overset{\|}{C}}-Aryl)$$

erfolgen.

Die Carbacyclin-Derivate der allgemeinen Formel I mit -$R_1$ in der Bedeutung einer Hydroxygruppe ($R_2$=H) können mit geeigneten Mengen der entsprechenden anorganischen Basen unter Neutralisierung in Salze überführt werden. Beispielsweise erhält man beim Lösen der entsprechenden Säuren in Wasser, welches die stöchiometrische Menge der Base enthält, nach Abdampfen des Wassers oder nach Zugabe eines mit Wasser mischbaren Lösungsmittels, zum Beispiel Alkohol oder Aceton, das feste anorganische Salz.

Die Herstellung der Aminsalze erfolgt in üblicher Weise. Dazu wird die Carbacyclin-Säure zum Beispiel in einem geeigneten Lösungsmittel, wie Äthanol, Aceton, Diäthyläther oder Benzol gelöst und mindestens die stöchiometrische Menge des Amins dieser Lösung zugesetzt. Dabei fällt das Salz gewöhnlich in fester Form an oder wird nach Verdampfen des Lösungsmittels in üblicher Weise isoliert.

Die funktionelle Abwandlung der freien OH-Gruppen erfolgt nach den dem Fachmann bekannten Methoden. Zur Einführung der Ätherschutzgruppen wird beispielsweise mit Dihydropyran in Methylenchlorid oder Chloroform unter Verwendung eines sauren Kondensationsmittels, wie zum Beispiel p-Toluolsulfonsäure, umgesetzt. Das Dihydropyran wird im Überschuß angewandt, vorzugsweise in der 4- bis 10-fachen Menge des theoretischen Bedarfs. Die Umsetzung ist normalerweise bei 0°C - 30°C nach 15 - 30 Minuten beendet.

Die Einführung der Acylschutzgruppen erfolgt, indem man eine Verbindung der allgemeinen Formel I in an sich bekannter Weise mit einem Carbonsäurederivat, wie zum Beispiel Säurechlorid, Säureanhydrid u.a., umsetzt.

Die Freisetzung einer funktionell abgewandelten OH-Gruppe zu den Verbindungen der allgemeinen Formel I erfolgt nach bekannten Methoden. Beispielsweise wird die Abspaltung von Ätherschutzgruppen in einer wäßrigen Lösung einer organischen Säure, wie zum Beispiel Essigsäure, Propionsäure u.a. oder in einer wäßrigen Lösung einer anorganischen Säure, wie zum Beispiel Salzsäure, durchgeführt. Zur Verbesserung der Löslichkeit wird zweckmäßigerweise ein mit Wasser mischbares inertes organisches Lösungsmittel zugesetzt. Geeignete organische Lösungsmittel sind zum Beispiel Alkohole, wie Methanol und Äthanol, und Äther, wie Dimethoxyäthan, Dioxan und Tetrahydrofuran. Tetrahydrofuran wird bevorzugt angewendet. Die Abspaltung wird vorzugsweise bei Temperaturen zwischen 20°C und 80°C durchgeführt.

Die Abspaltung der Silylätherschutzgruppen erfolgt beispielsweise mit Tetrabutylammoniumfluorid. Als Lösungsmittel sind beispielsweise geeignet Tetrahydrofuran, Diäthyläther, Dioxan, Methylenchlorid usw. Die Abspaltung wird vorzugsweise bei Temperaturen zwischen 0°C und 80°C durchgeführt.

Die Verseifung der Acylgruppen erfolgt beispielsweise mit Alkalioder Erdalkali-carbonaten oder -hydroxyden in einem Alkohol oder der wäßrigen Lösung eines Alkohols. Als Alkohole kommen aliphatische Alkohole in Betracht, wie zum Beispiel Methanol, Äthanol, Butanol usw., vorzugsweise Methanol. Als Alkalicarbonate und -hydroxyde seien Kalium- und Natriumsalze genannt, bevorzugt sind jedoch die Kaliumsalze. Als Erdalkalicarbonate und -hydroxyde sind beispielsweise geeignet Calciumcarbonat, Calciumhydroxyd und

Bariumcarbonat. Die Umsetzung erfolgt bei -10° C bis 70° C, vorzugsweise bei 25° C.

Die Einführung der Amidgruppe $NHR_3$ für $R_1$ erfolgt nach den dem Fachmann bekannten Methoden. Die Carbonsäuren der allgemeinen Formel I ($R_2=H$) werden zunächst in Gegenwart eines tertiären Amins, wie beispielsweise Triäthylamin mit Chlorameisensäureisobutylester in das gemischte Anhydrid überführt. Die Umsetzung des gemischten Anhydrids mit dem Alkalisalz des entsprechenden Amids oder mit Ammoniak ($R_3=H$) erfolgt in einem inerten Lösungsmittel oder Lösungsmittelgemisch, wie beispielsweise Tetrahydrofuran, Dimethoxyäthan, Dimethylformamid, Hexamethylphosphorsäuretriamid, bei Temperaturen zwischen -30° C und +60° C, vorzugsweise bei 0° C bis 30° C.

Eine weitere Möglichkeit für die Einführung der Amidgruppe $NHR_3$ für $R_1$ besteht in der Umsetzung einer 1-Carbonsäure der allgemeinen Formel I ($R_2=H$), in der freie Hydroxygruppen gegebenenfalls intermediär geschützt sind, mit Verbindungen der allgemeine, Formel IV,

$$O = C = N - R_3 \qquad \text{IV,}$$

worin $R_3$ die obenangegebene Bedeutung hat.

Die Umsetzung der Verbindung der allgemeinen Formel I ($R_1=OH$) mit einem Isocyanat der allgemeinen Formel IV erfolgt gegebenenfalls unter Zusatz eines tertiären Amins, wie z. B. Triäthylamin oder Pyridin. Die Umsetzung kann ohne Lösungsmittel oder in einem inerten Lösungsmittel, vorzugsweise Acetonitril, Tetrahydrofuran, Aceton, Dimethylacetamid, Methylenchlorid, Diäthyläther, Toluol, bei Temperaturen zwischen -80° C bis 100° C, vorzugsweise bei 0° C bis 30° C, vorgenommen werden.

Enthält das Ausgangsprodukt OH-Gruppen im Prostanrest, so werden diese OH-Gruppen auch zur Reaktion gebracht. Werden letztlich Endprodukte gewünscht, die freie Hydroxylgruppen im Prostanrest enthalten, geht man zweckmässigerweise von Ausgangsprodukten aus, in denen diese durch vorzugsweise leicht abspaltbare Äther- oder Acylreste intermediär geschützt sind.

Die als Ausgangsmaterial dienenden Verbindungen der allgemeinen Formel II können beispielsweise hergestellt werden, indem man in an sich bekannter Weise einen Aldehyd der Formel V (DE-OS-2 845 770)

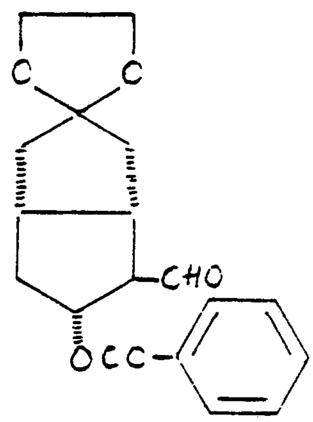

V,

mit einem Phosphonat der allgemeinen Formel VI

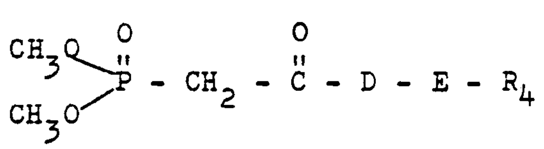

VI

worin D, E und $R_4$ die obenangegebene Bedeutung aufweisen, in Gegenwart eines Deprotonierungsmittels, wie beispielsweise Natriumhydrid oder Kalium-tert.-butylat zu einem Keton der allgemeinen Formel VII (X=H) oder zusätzlich in Gegenwart eines Bromierungsmittels, wie beispielsweise N-Bromsuccinimid, zu einem Keton der allgemeinen Formel VII (X=Br) umsetzt.

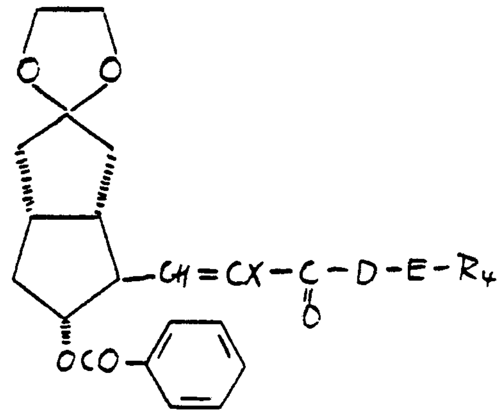

VII

Nach Reduktion der Ketogruppe mit Zinkborhydrid oder Natriumborhydrid oder Umsetzung mit Alkylmagnesiumbromid oder Alkyllithium und anschließender Epimerentrennung gelangt man zu den Verbindungen der allgemeinen Formel VIII

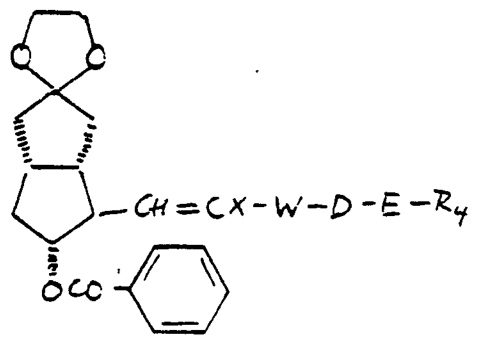

VIII

Verseifung der Estergruppe beispielsweise mit Kaliumcarbonat in Methanol sowie gegebenenfalls Hydrierung der Doppelbindung oder gegebenenfalls Verätherung mit Dihydropyran und anschließende Abspaltung von Bromwasserstoff mit beispielsweise Kalium-tert.butylat in Dimethylsulfoxid, Ketalspaltung mit wässriger Essigsäure sowie gegebenenfalls funktionelle Abwandlung der freien Hydroxygruppen, beispielsweise durch Verätherung mit Dihydropyran liefert das Keton der allgemeinen Formel IX.

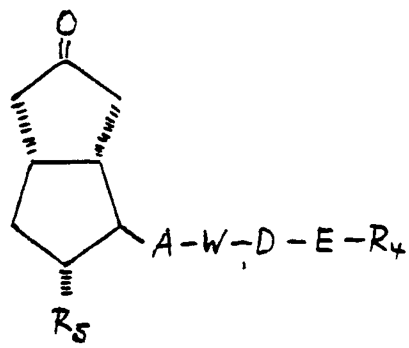

IX

Nach Olefinierungsreaktion mit Phosphonofluoressigsäuretriäthylester oder Phosphonofluoressigsäuretrimethylester und anschließender Reduktion mit Lithiumaluminiumhydrid erhält man die in der Doppelbindung isomeren Verbindungen der allgemeinen Formel II, die gegebenenfalls getrennt werden können.

Die Herstellung der Phosphonate der allgemeinen Formel VI erfolgt in an sich bekannter Weise durch Umsetzung des Anions von Methylphosphonsäuredimethylester mit einem Ester der allgemeinen Formel X

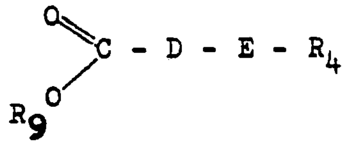

X

worin D, E, $R_4$ die obengenannten Bedeutungen aufweisen und $R_9$ eine Alkylgruppe mit 1-5 C-Atomen bedeutet, den man gegebenenfalls aus dem entsprechenden Malonsäureester durch Alkylierung mit dem Halogenid

Hal - E - $R_4$ XI

der allgemeinen Formel XI (mit Hal in der Bedeutung Chlor oder Brom) und anschließender Decarbalkoxylierung erhalten kann. Der Ester der allgemeinen Formel X ist gegebenenfalls auch aus der Carbonsäure der allgemeinen Formel XII

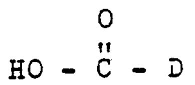

XII

worin D die obenangegebene Bedeutung aufweist durch Alkylierung mit dem Halogenid der allgemeinen Formel XI und anschließender Veresterung zugänglich.

Die Verbindungen dieser Erfindung wirken blutdrucksenkend und bronchodilatorisch. Sie sind weiterhin geeignet zur Hemmung der Thrombozyten-Aggregation. Folglich stellen die neuen Carbacyclin-Derivate der Formel I wertvolle pharmazeutische Wirkstoffe dar. Darüber hinaus weisen sie bei ähnlichem Wirkungsspektrum, verglichen mit entsprechenden Prostaglandinen, eine höhere Spezifität und vor allem eine wesentlich längere Wirksamkeit auf. Im Vergleich zu $PGI_2$ zeichnen sie sich durch größere Stabilität aus. Die hohe Gewebsspezifität der neuen Prostaglandine zeigt sich bei der Untersuchung an glattmuskulären Organen, wie z. B. am Meerschweinchenileum oder an der isolierten Kaninchentrachea, wo eine wesentlich geringere Stimulation zu beobachten ist als bei der Applikation natürlicher Prostaglandine vom E-, A- oder F-Typ.

Die neuen Carbacyclin-Analoga besitzen die für Prostacycline typischen Eigenschaften, wie z. B. Senkung des peripheren arteriellen und koronaren vaskulären Widerstandes, Inhibierung der Thrombozytenaggregation und Auflösung von Plättchenthromben, myocardiale Zytoprotektion und damit Senkung des systemischen Blutdruckes, ohne zugleich Schlagvolumen und koronare Durchblutung zu senken; Behandlung von Schlaganfall, Prophylaxe und Therapie koronarer Herzerkrankungen, koronarer Thrombose, des Herzinfarktes, peripherer Arterienerkrankungen, Arteriosklerose und Thrombose, Prophylaxe und Therapie ischaemischer Attacken des ZNS-Systems, Therapie des Schocks, Inhibierung der Bronchokonstriktion, Inhibierung der Magensäuresekretion, Zytoprotektion der Magen- und Darmschleimhaut, Zytoprotektion in der Leber und im Pankreas, anitallergische Eigenschaften, Senkung des pulmonaren vaskulären Widerstandes und des pulmonaren Blutdruckes, Förderung der Nierendurchblutung, Anwendung an Stelle von Heparin oder als Adjuvans bei der Dialyse der Hämofiltration, Konservierung von Blutplasmakonserven, besonders von Blutplättchenkonserven, Inhibierung von Geburtswehen, Behandlung von Schwangerschaftstoxikose, Erhöhung der zerebralen Durchblutung etc. Außerdem besitzen die neuen Carbacyclinderivate antiproliferative und antidiarrhoegene Eigenschaften. Die Carbacycline dieser Erfindung können auch in Kombination, z. B. mit: -Blockern oder Diuretika, verwendet werden.

Die Dosis der Verbindungen ist 1-1500 µg/kg/Tag, wenn sie am menschlichen Patienten verabreicht werden. Die Einheitsdosis für den pharmazeutischen akzeptablen Träger beträgt 0,01-100 mg.

Bei intravenöser Injektion an wachen, hypertonen Ratten in Dosen von 5, 20 und 100 µg/kg Körpergewicht zeigen die erfindungsgemäßen Verbindungen eine stärkere blutdrucksenkende und länger anhaltende Wirkung als $PGE_2$ und $PGA_2$, ohne wie $PGE_2$ Durchfälle oder $PGA_2$ kardiale Arrhythmien auszulösen.

Bei intravenöser Injektion an narkotisierten Kaninchen zeigen die erfindungsgemäßen Verbindungen in Vergleich zu $PGE_2$ und $PGA_2$ eine stärkere und herblich länger anhaltende Blutdrucksenkung, ohne daß andere glattmuskuläre Organe oder Organfunktionen beeinflußt werden.

Für die parenterale Verabreichung werden sterile, injizierbare, wässrige oder ölige Lösungen benutzt. Für die orale Applikation sind beispielsweise Tabletten, Dragees oder Kapseln geeignet.

Die Erfindung betrifft damit auch Arzneimittel auf Basis der Verbindungen der allgemeinen Formel I und üblicher Hilfs- und Trägerstoffe.

Die erfindungsgemäßen Wirkstoffe sollen in Verbindung mit den in der Galenik bekannten und üblichen Hilfsstoffen z. B. zur Herstellung von Blutdrucksenkern dienen.

**Beispiel 1**

**(5Z)-(16RS)-5-Fluor-16-methyl-3-oxa-18,18,19 19-tetradehydro-6a-carba-prostaglandin-$I_2$**

Zu einer Lösung von 310 mg 2-{(Z)-(1S,5S,6R,7R)-7-(Tetrahydropyran-2-yloxy)-6-[(E)-(3S,4RS)-4-methyl-3-(tetrahydropyran-2-yloxy)-oct-1-en-6-inyl]-bicyclo[3.3.0] octan-3-yliden}-2-fluor-äthan-1-ol in 10 ml Tetrahydrofuran gibt man 60,7 mg 55 %-ige Natriumhydridsuspension in Mineralöl und rührt eine Stunde unter Argon. Nach Abkühlen auf 0°C tropft man im Verlauf von einer Stunde eine Lösung von 96,6 mg Bromessigsäure in 3 ml Tetrahydrofuran zu und rührt anschließend 26 Stunden bei 20°C, verdünnt mit 150 ml Äther und schüttelt viermal mit je 50 ml 4 %-iger Natronlauge. Dieser Extrakt wird mit 10 %-iger Schwefelsäure bei 0°C auf pH3 eingestellt und viermal mit je 50 ml Dichlormethan extrahiert. Der organische Extrakt wird mit 20 ml Sole geschüttelt, über Magnesiumsulfat getrocknet und im Vakuum eingedampft. Man erhält 305 mg (5Z)-(16RS)-5-Fluor-16-methyl-3-oxa-18,18,19,19-tetradehydro-6a-carba-prostaglandin-$I_2$-11,15-bis-(tetrahydropyranyläther), die man zur Abspaltung der Schutzgruppen 18 Stunden mit 20 ml Essigsäure/Wasser/Tetrahydrofuran (65/35/10) rührt. Man dampft unter Zusatz von Toluol ein und chromatographiert den Rückstand an Kieselgel mit Essigester/0,1 - 1 % Essigsäure und erhält 102 mg der Titelverbindung als farbloses Öl.

IR: 3600, 3400 (breit), 2955, 2920, 1731, 1601, 1108, 970/cm.

Das Ausgangsmaterial für die obige Titelverbindung wird wie folgt hergestellt:

1 a) <u>2-{(Z)-(1S,5S,6R,7R)-7-(Tetrahydropyran-2-yloxy)-6-[(E)-(3S,4RS)-4-methyl-3-(tetrahydropyran-2-yloxy)oct-1-en-6-inyl]-bicyclo [3.3.0] octan-3-yliden}-2-fluor-äthan-1-ol</u>

Man suspendiert 128,4 mg Natriumhydrid (55 %-ige Mineralölsuspension) in 4 ml Dimethoxyäthan, tropft bei 0°C unter Argon eine Lösung von 791,5 mg Phosphonofluoressigsäuretriäthylester in 2 ml Dimethoxyäthan zu und rührt 1,5 Stunden bei 20°C, tropft dann eine Lösung von 775 mg (1R,5S,6R,7R)-7-(Tetrahydropyran-2-yloxy)-6-[(E)-(3S,4RS)-4-methyl-3-(tetrahydropyran-2-yloxy)-oct-1-en-6-inyl]-bicyclo[3.3.0]octan-3-on in 2 ml Dimethoxyäthan zu und rührt 22 Stunden. Dann wird mit 8 ml Wasser versetzt und dreimal mit je 50 ml Äther ausgeschüttelt, der Extrakt über Magnesiumsulfat getrocknet und im Vakuum eingedampft. Man erhält 1,30 g öliges Produkt, das man zur Reinigung an Kieselgel mit Hexan/Äther (1+1) chromatographiert. Dabei erhält man 500 mg öligen Ester, den man zur Reduktion in 20 ml Äther löst und bei 0°C für 1 Stunde mit 180 mg Lithiumaluminiumhydrid rührt. Man versetzt dann mit 0,75 ml Wasser, rührt 2 Stunden bei 20°C filtriert und dampft im Vakuum ein. Den Rückstand reinigt man durch Chromatographie an Kieselgel mit Hexan/10-90 % Äther. Zunächst eluiert man als unpolarere Komponente 195 mg 2-{(E)-(1S,5S,6R,7R)-7-(Tetrahydropyran-2-yloxy)-6-[(E)-(3S,4RS)-4-methyl-3-(tetrahydropyran-2-yloxy)-oct-1-en-6-inyl]-bicyclo[3.3.0]octan-3-yliden}-2-fluor-äthan-1-ol und als polarere Komponente 198 mg der Titelverbindung, beide als Öl.

IR: 3600, 3425, 2940, 1161, 970/cm

**Beispiel 2**

**(5E)-(16RS)-5-Fluor-16-methyl-3-oxa-18,18,19,19-tetradehydro-6a-carba-prostaglandin-$I_2$**

In Analogie zu Beispiel 1 erhält man aus 180 mg 2-{(E)-(1S,5S,6R,7R)-7-(Tetrahydropyran-2-yloxy)-6-[(E)-(3S,4RS)-4-methyl-3-(tetrahydropyran-2-yloxy)-oct-1-en-6-inyl]-bicyclo[3.3.0]octan-3-yliden}-2-fluor-äthan-1-ol 45 mg der Titelverbindung als farbloses Öl.

IR: 3600, 3400 (breit), 2958, 2922, 1732, 1600, 1110, 974/cm

**Beispiel 3**

**(5Z)-(16RS)-16,20-Dimethyl-5-fluor-3-oxa-18,18,19,19-tetradehydro-6a-carba-prostaglandin-$I_2$**

In Analogie zu Beispiel 1 erhält man aus 400 mg 2-{(Z)-(1S,5S,6R,7R)-7-(Tetrahydropyran-2-yloxy)-6-[(E)-(3S,4RS)-4-methyl-3-(tetrahydropyran-2-yloxy)-non-1-en-6-inyl]-bicyclo[3.3.0]octan-3-yliden)-2-fluor-äthan-1-ol 125 mg der Titelverbindung als farbloses Öl.

IR: 3600, 3405 (br.), 2954, 2920, 1730, 1601, 1115, 970/cm

Das Ausgangsmaterial für die obige Titelverbindung wird wie folgt hergestellt:

3 a) <u>2-{(Z)-(1S 5S,6R,7R)-7-(Tetrahydropyran-2-yloxy)-6-[(E)-(3S,4RS)-4-methyl-3-(tetrahydropyran-2-yloxy)-non-1-en-6-inyl]-bicyclo[3.3.0]octan-3-yliden}2-fluor-äthan-1-ol</u>

In Analogie zu Beispiel 1a erhält man aus 1 g (1R,5S,6R,7R)-7-(Tetrahydropyran-2-yloxy)-6-[(E)-(3S,4RS)-4-methyl-3-(tetrahydropyran-2-yloxy)-non-1-en-6-inyl]-bicyclo[3.3.0]octan-3-on nach chromatographischer

Isomerentrennung als unpolare Verbindung 235 mg 2-{(E)-(1S,5S,6R,7R)-7-(Tetrahydropyran-2-yloxy-6-[(E)-(3S,4RS)-4-methyl-3-(tetrahydropyran-2-yloxy)-non-1-en-6-inyl]-bicyclo[3.3.0]octan-3-yliden}-2 fluor-äthan-1-ol und 245 mg der Titelverbindung als farbloses Öl.

IR: 3600, 3422, 2945, 1165, 972/cm

**Beispiel 4**

**(5Z)-5-Fluor-20-methyl-3-oxa-18,18,19,19-tetradehydro-16,16-trimethylen-6a-carba-prostaglandin-$I_2$**

In Analogie zu Beispiel 1 erhält man aus 200 mg 2- {(Z)-(1S,5S,6R,7R)-7-(Tetrahydropyran-2-yloxy)-6-[(E)-(3R)-3-(tetrahyrdopyran-2-yloxy)-4,4-trimethylen-non-1-en-6-inyl]-bicyclo[3.3.0]octan-3-yliden}-2-fluor-äthan-1-ol 58 mg der Titelverbindung als farbloses Öl.

IR: 3600, 3405 (br.), 2952, 2915, 1734, 1601, 1110, 970/cm

Das Ausgangsmaterial für die obige Titelverbindung wird wie folgt hergestellt:

4 a) 2-{(Z)-(1S,5S,6R,7R)-Z-(Tetrahydropyran-2-yloxy)-6-[(E)-(3R)-3-(tetrahydropyran-2-yloxy)-4,4-trimethylen-non-1-en-6-inyl]-bicyclo[3.3.0]octan-3-yliden}-2-fluor-äthan-1-ol

In Analogie zu Beispiel 1a erhält man aus 1 g (1R,5S,6R,7R)-7-(Tetrahydropyran-2-yloxy)-6-[(E)-(3R)-3-(tetrahydropyran-2-yloxy)-4,4-trimethylen-non-1-en-6-inyl]-bicyclo[3.3.0]octan-3-on- nach chromatographischer Isomerentrennung als unpolare Verbindung 220 mg 2- {(E)-(1S,5S,6R,7R)-7-(tetrahydropyran-2-yloxy)-6-[(E)-(3R)-3-(tetrahydropyran-2-yloxy)-4-trimethylen-non-1-en-6-inyl]-bicyclo[3.3.0]octan-3-yliden}-2-fluor-äthan-1-ol und 240 mg der Titelverbindung als farbloses Öl.

IR: 3600, 3410, 2945, 1160, 972/cm

**Beispiel 5**

**(5Z)-16,16-Dimethyl-5-fluor-3-oxa-18,18,19,19-tetradehydro-6a-carba-prostaglandin-$I_2$**

>r In Analogie zu Beispiel 1 erhält man aus 420 mg 2-{(Z)-(1S,5S,6R,7R)-7-(Tetrahydropyran-2-yloxy)6-[(E)-(3R)-4,4-dimethyl-3-(tetrahydropyran-2-yloxy)-oct-1-en-6-inyl]-bicyclo[3.3.0]octan-3-yliden}-2-fluor-äthan-1-ol 140 mg der Titelverbindung als farbloses Öl.

IR: 3600, 3400 (br.), 2958, 2921, 1732, 1601, 1110, 976/cm.

Das Ausgangsmaterial für die obige Titelverbindung wird wie folgt hergestellt:

5 a) 2-{(Z)-(1S,5S,6R,7R)-7-(Tetrahydropyran-2-yloxy)-6-[(E)-(3R)-4,4-dimethyl-3-(tetrahydropyran-2-yloxy)-oct-1-en-6-inyl]-bicyclo[3.3.0]octan-3-yliden}-2-fluor-äthan-1-ol

In Analogie zu Beispiel 1a erhält man aus 800 mg (1R,5S,6R,7R)-7-(Tetrahydropyran-yloxy)-6-[(E)-(3R)-4,4-dimethyl-3-(tetrahydropyran-2-yloxy)-oct-1-en-6-inyl]-bicyclo[3.3.0]octan-3-on nach chromatographischer Isomerentrennung als unpolare Verbindung 178 mg 2-{(E)-(1S,5S,6R,7R)-7-(Tetrahydropyran-2-yloxy)-6-[(E)-(3R)-4,4-dimethyl-3-(tetrahydropyran-2-yloxy)-oct-1-en-6-inyl]-bicyclo[3.3.0]octan-3-yliden}-2-fluor-äthan-1-ol und 188 mg der Titelverbindung als farbloses Öl.

IR: 3600, 3405, 2950, 1162, 976/cm

**Beispiel 6**

**(5Z)-5-Fluor-3-oxa-16,16,20-trimethyl-18,18,19,19-tetradehydro-6a-carba-prostaglandin-$I_2$**

In Analogie zu Beispiel 1 erhält man aus 400 mg 2-{(Z)-(1S,5S,6R,7R)-7-(Tetrahydropyran-2-yloxy)-6-[(E)-(3R)-4,4-dimethyl-3-(tetrahydropyran-2-yloxy)-non-1-en-6-inyl]-bicyclo[3.3.0]octan-3-yliden}-2-fluor-äthan-1-ol 128 mg der Titelverbindung als farbloses Öl.

IR: 3600, 3405 (br.), 2960, 2922, 1735, 1601, 1116, 976/cm

Das Ausgangsmaterial für die obige Titelverbindung wird wie folgt hergestellt:

6 a) 2-{(Z)-(1S,5S,6R,7R)-7-(Tetrahydropyran-2-yloxy)-6-[(E)-(3R)-4,4-dimethyl-3-(tetrahydropyran-2-yloxy)-non-1-en-6-inyl]-bicyclo[3.3.0]octan-3-yliden}-2-fluor-äthan-1-ol

>r In Analogie zu Beispiel 1a erhält man aus 2 g (1R,5S,6R,7R)-7-(Tetrahydropyran-2-yloxy)-6-[(E)-(3R)-4,4-dimethyl-3-(tetrahydropyran-2-yloxy)-non-1-en-6-inyl]-bicyclo[3.3.0]octan-3-on nach chromatographischer Isomerentrennung als unpolarere Verbindung 420 mg 2-{(E)-(1S,5S,6R,7R)-7-(Tetrahydropyran-2-yloxy)-6-[(E)-(3R)-4,4-dimethyl-3-(tetrahydropyran-2-yloxy)-non-1-en-6-inyl]-bicyclo[3.3.0]octan-3-yliden}-2-fluor-äthan-1-ol und 450 mg der Titelverbindung als farbloses Öl.
IR: 3600, 3410, 2952, 1160, 974/cm

**Beispiel 7**

**(5Z)-(16RS)-16,19-Dimethyl-5-fluor-18,19-didehydro-3-oxa-6a-carba-prostaglandin-$I_2$**

In Analogie zu Beispiel 1 erhält man aus 150 mg 2-{(Z)-(1S,5S,6R,7R)-7-(Tetrahydropyran-2-yloxy)-6-[(E)-(3S,4RS)-4,7-dimethyl-3-(tetrahydropyran-2-yloxy)-octa-1,6-dienyl]-bicyclo[3.3.0]octan-3-yliden}-2-fluor-äthan-1-ol 48 mg der Titelverbindung als farbloses Öl.
IR: 3600, 3410 (br.), 2958, 2930, 1730, 1601, 1110, 976/cm
Das Ausgangsmaterial für die obige Titelverbindung wird wie folgt hergestellt:

7 a) 2-{(Z)-(1S,5S,6R,7R)-7-(Tetrahydropyran-2-yloxy)-6-[(E)-(3R,4RS)-4,7-dimethyl-3-(tetrahydropyran-2-yloxy)-octa-1,6-dienyl]-bicyclo [3.3.0]octan-3-yliden}-2-fluor-äthanl-ol

In Analogie zu Beispiel 1a erhält man aus 1 g (1R,5S,6R,7R)-7-(Tetrahydropyran-2-yloxy)-6-[(E)-(3R,4RS)-4,7-dimethyl-3-(tetrahydropyran-2-yloxy)-octa-1,6-dienyl]-bicyclo[3.3.0]ctan-on nach chromatographischer Trennung als unpolarere Verbindung 240 mg 2-{(E)-(1S,5S,6R,7R)-7-(Tetrahydropyran-2-yloxy)-6-[(E)-(3S,4RS)-4,7-dimethyl-3-(tetrahydropyran-2-yloxy)-octa-1,6-dienyl]-bicyclo[3.3.0]octan-3-yliden}-2-fluor-äthan-1-ol und 250 mg der Titelverbindung als farbloses Öl.
IR: 3600, 3408, 2948, 1161, 970/cm

**Beispiel 8**

**(5Z)-(16RS)-13,14-Didehydro-5-fluor-16-methyl-3-oxa-18,18,19,19-tetradehydro-6a-carba-prostaglandin-$I_2$**

Zu einer Lösung von 300 mg 2-{(Z)-(1S,5S,6R,7R)-7-(Tetrahydropyran-2-yloxy)-6-[(3S,4RS)-4-methyl-3-(tetrahydropyran-2-yloxy)-octa-1,6-diinyl]-bicyclo[3.3.0]octan-3-yliden}-2-fluor-äthan-1-ol in 10 ml Tetrahydropyran gibt man 58,5 mg Natriumhydrid (55 %-ige Suspension in Mineralöl), ruhrt 1 Stunde und tropft bei 0°C im Verlauf von 1 Stunde eine Lösung von 94 mg Bromessigsäure in 3 ml Tetrahydrofuran zu und rührt anschließend 24 Stunden bei 20°C, verdünnt mit 150 ml Äther und schüttelt viermal mit je 50 ml 4 %-ige Natronlauge aus. Dieser Extrakt wird mit 10 %-iger Schwefelsäure bei 0°C auf pH3 eingestellt und viermal mit je 50 ml Dichlormethan extrahiert. Die vereinigten Dichlormethanextrakte werden mit 20 ml Sole geschüttelt, über Magnesiumsulfat getrocknet und im Vakuum eingedampft. Das Rohprodukt wird 18 Stunden mit 20 ml Essigsäure/Wasser/Tetrahydrofuran (65/35/10) gerührt, unter Zusatz von Toluol eingedampft und an Kieselgel chromatographiert. Mit Essigester/0,3 % Essigsäure eluiert man 110 mg der Titelverbindung als farbloses Öl.
IR: 3600, 3420 (br.), 2942, 2918, 1730, 1115/cm
Das Ausgangsmaterial für die obige Titelverbindung wird wie folgt hergestellt:

8 a) (1R,5S,6S,7R)-3,3-Äthylendioxy-7-benzoyloxy-6-[(4RS)-2-brom-4-methyl-3-oxo-oct-1-en-6-inyl]-bicyclo[3.3.0]octan

Zu einer Suspension von 1,81 g Natriumhydrid in 180 ml Dimethoxyäthan tropft man bei 0°C eine Lösung von 10,5 g 3-Methyl-2-oxo-hept-5-in-phosphonsäuredimethylester in 70 ml Dimethoxyäthan, rührt 1 Stunde bei 0°C und fügt dann 7,4 g fein gepulvertes N-Bromsuccinimid hinzu. Man rührt 30 Minuten bei 0°C, versetzt mit einer Lösung von 11,4 g (1R,5S,6R,7R)-3,3-Äthylendioxy-7-benzoyloxy-6-formyl-bicyclo[3.3.0]octan in 90 ml Dimethoxyäthan und rührt 2 Stunden bei 0°C. Das Reaktionsgemisch gießt man auf gesättigte Ammoniumchloridlösung und extrahiert mit Äther. Man wäscht den organischen Extrakt mit Wasser neutral, trocknet über Magnesiumsulfat und dampft im Vakkum ein. Nach Chromatographie des Rückstandes an Kieselgel erhält man mit Hexan/Äther (3 + 2) 8,2 g des ungesättigten Ketons als farbloses Öl.
IR: 2930, 2880, 1712, 1688, 1602, 1595, 1450, 1275, 945/cm

8 b) (1R,3S,6S,7R)-3,3-Äthylendioxy-7-(tetrahydrobyran-2-yloxy)-6-[(3S,4RS)-2-brom-4-methyl-3-(tetrahydropyran-2-yloxy)-oct-1-en-6-inyl]-bicyclo[3.3.0]octan

Zu einer Lösung von 5,9 g des nach Beispiel 8a hergestellten Ketons in 140 ml Methanol fügt man bei -40°C portionsweise 2,5 g Natriumborhydrid und rührt 30 Minuten bei -40°C. Anschließend verdünnt man mit Äther, wäscht mit Wasser neutral, trocknet über Magnesiumsulfat und dampft im Vakuum ein. Das Rohprodukt (15-Epimerengemisch) löst man in 200 ml Methanol, fügt 2,5 g Kaliumcarbonat zu und rührt 17 Stunden bei 23°C unter Argon. Anschließend engt man im Vakuum ein, verdünnt mit Äther und wäscht mit Sole neutral. Man trocknet über Magnesiumsulfat und dampft im Vakuum ein. Durch Säulenchromatographie an Kieselgel mit Äther/Methylenchlorid erhält man zunächst 1,6 g des 15ß-konfigurierten Alkohols sowie als polarere Komponente 2,1 g der Titelverbindung (pG-Nomenklatur 15 α-Hydroxy) als farblose Öle. Eine Lösung aus 1,6 g des α-Alkohols, 16 mg p-Toluolsulfonsäure und 1,5 g Dihydropyran in 50 ml Methylenchlorid rührt man 35 Minuten bei 0°C. Anschließend verdunnt man mit Äther, schüttelt mit verdünnter Natriumcarbonatlösung, wäscht mit Wasser neutral, trocknet über Magnesiumsulfat und dampft im Vakuum ein. Nach Chromatographie des Rückstandes an Kieselgel erhält man mit Hexan/Äther (7+3) 2,17 g der Titelverbindung als farbloses Öl.

IR: 2940, 2870, 1450, 1120, 1018, 965, 948/cm

8 c) (1R,5S,6S,7R)-7-(Tetrahydropyran-2-yloxy)-6-[(3S,4RS)-4-methyl-3-(tetrahydropyran-2-yloxy)-octa-1,6-diinyl]-bicyclo [3.3.0]octan-3-on

Eine Lösung von 2,30 g der nach Beispiel 8 b hergestellten Verbindung in 23 ml Dimethylsulfoxid und 10 ml Tetrahydrofuran gibt man 667 mg Kalium-tert.Butylat und rührt 2 Stunden bei 20°C. Man verdünnt mit 100 ml Wasser und extrahiert dreimal mit je 100 ml Ather/Hexan (8+2), wäscht den Extrakt mit je 30 ml Wasser und Sole, trocknet über Magnesiumsulfat und dampft im Vakuum ein. Man rührt den Rückstand 22 Stunden mit 75 ml Essigsäure/Wasser/Tetrahydrofuran (65/35/10), dampft im Vakuum ein und reinigt den Rückstand durch Chromatographie an Kieselgel. Mit Äther eluiert man 1,05 g ölige Substanz, die man in 40 ml Dichlormethan mit 0,91 g Dihydropyran und 10 mg p-Toluolsulfonsäure bei 0°C umsetzt. Nach 30 Minuten verdünnt man mit Äther, schüttelt mit Natriumhydrogencarbonatlösung und Sole, trocknet über Magnesiumsulfat und dampft im Vakuum ein. Nach Chromatographie an Kieselgel mit Hexan/Äther (1 : 1) erhält man 1,53 g der Titelverbindung als farbloses Öl.

IR: 2942, 2876, 2210, 1737, 1018, 970, 905, 868/cm

8 d) 2-{(Z)-(1S,5S,6S,7R)-7-(Tetrahydropyran-2-yloxy)-6-[(3S,4RS)-4-methyl-3-(tetrahydropyran-2-yloxy)-octa-1,6-diinyl]-bicyclo [3.3.0]octan-3-yliden}-2-fluor-äthan-1-ol

Zu einer Suspension von 130 mg Natriumhydrid (55 %-ig) in 6 ml Dimethoxyäthan gibt man 800 mg Phosphonfluoressigsäuretriäthylester. Man rührt 1 Stunde und tropft dann eine Lösung von 800 mg des nach Beispiel 8 c hergestellten Ketons in 3 ml Dimethoxyäthan zu und ruhrt über Nacht bei 20°C. Nach Versetzen mit 10 ml Wasser extrahiert man dreimal mit je 50 ml Äther, trocknet den Extrakt über Magnesiumsulfat und dampft im Vakuum ein. Den öligen Rückstand löst man in 30 ml wasserfreien Äther, versetzt protionsweise bei 0° mit 250 mg Lithiumaluminiumhydrid, rührt 1 Stunde und versetzt tropfenweise mit 1 ml Wasser, dann mit 100 ml Äther. Man rührt noch 1 Stunde bei 20°C, filtriert und dampft im Vakuum ein. Den Rückstand reinigt man durch Chromatographie an Kieselgel mit Hexan/20-80 % Äther. Zunächst eluiert man als unpolarere Verbindung 200 mg 2-{(E)-(1S,5S,6S,7R)-7-(Tetrahydropyran-2-yloxy)-6-[(3S,4RS)-4-methyl-3-(tetrahydropyran-2-yloxy)-octa-1,6-diinyl]-bicyclo[3.3.0]octan-3-yliden}-2-fluor-äthan-1-ol und als polarere Verbindung 210 mg der Titelverbindung als farbloses Öl.

IR: 3600, 3440, 2941, 2215, 1132, 1018, 971, 905, 865/cm

**Beispiel 9**

**(5Z)-(16RS)-13,14-Didehydro-16,20-dimethyl-5-fluor-3-oxa-18,18,19,19-tetradehydro-6a-carba-prostaglandin-$I_2$**

In Analogie zu Beispiel 8 erhält man aus 200 mg 2-{(Z)-(1S,5S,6S,7R)-7-(Tetrahydropyran-2-yloxy)-6-[(3S,4RS)-4-methyl-3-(tetrahydropyran-2-yloxy)-nona-1,6-diinyl]-bicyclo[3.3.0]octan-3-yliden}-2-fluor-äthan-1-ol 80 mg der Titelverbindung als farbloses Öl.

IR: 3600, 3418 (br.), 2951, 2922, 1730, 1118/cm

Das Ausgangsmaterial für die obige Titelverbindung wird wie folgt hergestellt:

9 a) (1R,5S,6S,7R)-3,3-Äthylendioxy-7-benzoyloxy-6-[(4RS)-2-brom-4-methyl-3-oxo-non-1-en-6-inyl]-bicyclo[3.3.0.]octan

In Analogie zu Beispiel 8 a erhält man unter Verwendung von 3-Methyl-2-oxo-oct-5-in-phosphonsäuredimethylester aus 12 g des dort eingesetzten Aldehyds 8,80 g der Titelverbindung als farbloses Öl.

IR: 2935, 2880, 1715, 1601, 1593, 1451, 1270, 948/cm

9 b) (1R,5S,6S,7R)-3,3-Äthylendioxy-7-(tetrahydropyran-2-yloxy)-6-[(3S,4RS)-2-brom-4-methyl-3-(tetrahydropyran-2-yloxy)-non-1-en-6-inyl]-bicyclo[3.3.0]octan

In Analogie zu Beispiel 8 b erhält man aus 8,50 g des nach Beispiel 9 a erhaltenen ungesättigten Ketons 3,65 g der Titelverbindung als farbloses Öl.
IR: 2938, 2878, 1450, 968, 948/cm

9 c) (1R,5S,6S,7R)-7-(Tetrahydropyran-2-yloxy)-6-[(3S,4RS)-4-methyl-3-(tetrahydropyran-2-yloxy)-nona-16-diinyl]-bicyclo[3.3.0]ocyan-3-on

In Analogie zu Beispiel 8 c erhält man aus 3,50 g der nach Beispiel 9 b hergestellten Substanz 2,41 g der Titelverbindung als farbloses Öl.
IR: 2940, 2875, 2218, 1738, 1020, 970, 906, 870/cm

9 d) 2-{(Z)-(1S,5S,6S,7R)-7-(Tetrahydropyran-2-yloxy)-6-[(3S,4RS)-4-methyl-3-(tetrahydropyran-2-yloxy)-nona-16-diinyl]-bicyclo [3.3.0]octan-3-yliden}-2-fluor-äthan-1-ol

In Analogie zu Beispiel 8 d erhält man aus 2 g des nach Beispiel 9 c hergestellten Ketons nach chromatographischer Isomerentrennung 490 mg unpolareres 2-{(E)-(1S,5S,6S,7R)-7-(Tetrahydropyran-2-yloxy)-6-[(3S,4RS)-4-methyl-3-(tetrahydropyran-2-yloxy)-nona-1,6-diinyl]-bicyclo[3.3.0]octan-3-yliden}-2-fluor-äthan-1-ol und als polarere Komponente 505 mg der Titelverbindung als farbloses Öl.
IR: 3600, 3435, 2940, 2210, 1130, 1015, 970, 905, 868/cm

**Beispiel 10**

**(5Z)-13,14-Didehydro-5-fluor-20-methyl-3-oxa-18,18,19,19-tetradehydro-16,16-trimethylen-6a-carba-prostaglandin-$I_2$**

In Analogie zu Beispiel 8 erhält man aus 200 mg 2-{(Z)-(1S,5S,6S,7R)-7-(Tetrahydropyran-2-yloxy)-6-[(3S)-3-(tetrahydropyran-2-yloxy)-4,4-trimethylen-nona-1,6-diinyl]-bicyclo[3.3.0]octan-3-yliden)-2-fluor-äthan-1-ol 90 mg der Titelverbindung als farbloses Öl.
IR: 3600, 3420 (br.), 2950, 2920, 1732, 1116/cm
Das Ausgangsmaterial für die obige Titelverbindung wird wie folgt hergestellt:

10 a) (1R,3S,6S,7R)-3,3-Äthylendioxy-7-benzoyloxy-6-(2-brom-3-oxo-4,4-trimethylen-non-1-en-6-inyl)-bicyclo[3.3.0]octan

In Analogie zu Beispiel 8 a erhält man unter Verwendung von 2-Oxa-3,3-trimethylen-oct-5-in-phosphonsäuredimethylester aus 10 g des dort eingesetzten Aldehyds 9,01 g der Titelverbindung als farbloses Öl.
IR: 2938, 2880, 1712, 1690, 1600, 1592, 1451, 1273, 948/cm

10 b) (1R,5S,6S,7R)-3,3-Äthylendioxy-7-(tetrahydropyran-2-yloxy)-6-[(3S)-2-brom-3-(tetrahydropyran-2-yloxy)-4,4-trimethylen-non-1-en-6-inyl]-bicyclo[3.3.0]octan

In Analogie zu Beispiel 8 b erhält man aus 9 g der nach Beispiel 10 a hergestellten Verbindung 3,40 g der Titelverbindung als farbloses Öl.
IR: 2940, 2880, 1451, 970, 948/cm

10 c) (1R,5S,6S,7R)-7-(Tetrahydropyran-2-yloxy)-6-[(3S)-3-(tetrahydropyran-2-yloxy)-4,4-trimethylen-nona-1,6-diinyl]-bicyclo [3.3.0]octan-3-on

In Analogie zu Beispiel 8 c erhält man aus 3,31 g der nach Beispiel 10 b hergestellten Verbindung 2,05 g der Titelverbindung als farbloses Öl.
IR: 2945, 2873, 2210, 1736, 1015, 970, 906, 869/cm

10 d) 2-{(Z)-(1S,5S,6S,7R)-7-(tetrahydropyran-2-yloxy)-6-[(3S)-3-(tetrahydropyran-2-yloxy)-4,4,-trimethylen-nona-1,6-diinyl]-bicyclo[3.3.0]octan-3-yliden}-2-fluor-äthan-1-ol

In Analogie zu Beispiel 8 d erhält man aus 2 g des nach Beispiel 10 c hergestellten Ketons nach chromatographischer Trennung 430 mg unpolareres 2-{(E)-(1S,5S,6S,7R)-7-(Tetrahydropyran-2-yloxy)-6-[(3S)-3-(tetrahydropyran-2-yloxy)-4,4-trimethylen-nona-1,6-diinyl]-bicyclo[3.30]octan-3-yliden}-2-fluor-äthan-1-ol und als polarere Komponente 505 mg der Titelverbindung als farbloses Öl.
IR: 3600, 3420, 2942, 2215, 1135, 1013, 970, 908, 870/cm

**Beispiel 11**

**(5Z)-13,14-Didehydro-16,16-dimethyl-5-fluor-3-oxa-18,18,19,19-tetradehydro-6a-carba-prostaglandin-$I_2$**

In Analogie zu Beispiel 8 erhält man aus 180 mg 2-{(Z)-(1S,5S,6S,7R)-7-(Tetrahydropyran-2-yloxy)-6-[(3S)-4,4-dimethyl-3-(tetrahydropyran-2-yloxy)-octa-1,6-diinyl]-bicyclo[3.3.0]octan-3-yliden}-2-fluor-äthan-1-ol 85 mg der Titelverbindung als farbloses Öl.
IR: 3600, 3420 (br.), 2948, 2922, 1731, 1120/cm

11 a) (1R,5S,6S,7R)-3,3-Äthylendioxy-7-benzoyloxy-6-(2-brom-4,4-dimethyl-3-oxo-oct-1-en-6-inyl)-bicyclo[3.3.0]octan

In Analogie zu Beispiel 8 a erhält man unter Verwendung von 3,3-Dimethyl-2-oxo-hept-5-in-phosphonsäuredimethylester und 10 g des dort eingesetzten Aldehyds 7,90 g der Titelverbindung als farbloses Öl.
IR: 2935, 2875, 1710, 1685, 1601, 1594, 1450, 1270, 947/cm

11 b) (1R,5S,6S,7R)-3,3-Äthylendioxy-7-(tetrahydropyran-2-yloxy)-6-[(3S)-2-brom-4,4-dimethyl-3-(tetrahydropyran-2-yloxy)-oct-1-en-6-inyl]-bicyclo[3.3.0]octan

In Analogie zu Beispiel 8 b erhält man aus 7,85 g des nach Beispiel 11 a erhaltenen ungesättigten Ketons 3,32 g der Titelverbindung als farbloses Öl.
IR: 2940, 2877, 1451, 970, 948/cm

11 c) (1R,5S,6S,7R)-7-(Tetrahydropyran-2-yloxy-6-[(3S)-4,4-dimethyl-3-(tetrahydropyran-2-yloxy)-octa-1,6-diinyl]-bicyclo[3.3.0]octan -3-on

In Analogie zu Beispiel 8 c erhält man aus 3,21 g der nach Beispiel 11 b hergestellten Substanz 2,05 g der Titelverbindung als farbloses Öl.
IR: 2945, 2875, 2213, 1740, 1025, 970, 906, 871/cm

11 d) 2-{(Z)-(1S,5S,6S,7R)-7-(Tetrahydropyran-2-yloxy)-6-[(3S)-4,4-dimethyl-3-(tetrahydropyran-2-yloxy)-octa-1,6-diinyl]-bicyclo[3.3.0]octan-3-yliden}-2-fluor-äthan-1-ol

In Analogie zu Beispiel 8 d erhält man aus 2 g des nach Beispiel 11 c hergestellten Ketons nach chromatographischer Isomerentrennung 480 mg unpolareres 2-{(E)-(1S,5S,6S,7R)-7-(Tetrahydropyran-2-yloxy)-6-[(3S)-4,4-dimethyl-3-(tetrahydropyran-2-yloxy)-octa-1,6-diinyl]-bicyclo[3.3.0]octan-3-yliden} -2-fluor-äthan-1-ol und als polarere Komponente 525 mg der Titelverbindung als farbloses Öl.
IR: 3600, 3430, 2940, 2212, 1135, 1021, 970, 908, 871/cm

**Beispiel 12**

**(5Z)-13,14-Didehydro-5-fluor-3-oxa-18,18,19,19-tetradehydro-16,16,20-trimethyl-6a-carba-prostaglandin-$I_2$**

In Analogie zu Beispiel 8 erhält man aus 150 mg 2-{(Z)-(1S,5S,6S,7R)-7-(Tetrahydropyran-2-yloxy-)-6-[(3S)-4,4-dimethyl-3-(tetrahydropyran-2-yloxy)-nona-1,6-diinyl]-bicyclo[3.3.0]octan-3-yliden)-2-fluor-äthan-1-ol 68 mg der Titelverbindung als farbloses Öl.
IR: 3600, 3415 (br.), 2951, 2925, 1731, 1128/cm

12 a) (1R,5S,6S,7R)-3,3-Äthylendioxy-7-benzoyloxy-6-(2-brom-4,4-dimethyl-3-oxo-non-1-en-6-inyl)-bicyclo[3.3.0]octan

In Analogie zu Beispiel 8 a erhält man unter Verwendung von 3,3-Dimethyl-2-oxo-oct-5-in-phosphonsäuredimethylester und 12 g des in Beispiel 8 a eingesetzten Aldehyds 8,10 g der Titelverbindung als farbloses Öl.
IR: 2938, 2882, 1711, 1685, 1600, 1593, 1450, 1273, 948/cm

12 b) (1R,5S,6S,7R)-3,3-Äthylendioxy-7-(tetrahydropyran-2-yloxy)-6-[(3S)-2-brom-4,4-dimethyl-3-(tetrahydropyran-2-yloxy)-non-1-en-6-inyl]-bicyclo[3.3.0]octan

In Analogie zu Beispiel 8 b erhält man aus 8,00 g des nach Beispiel 12 a erhaltenen ungesättigten Ketons 3,51

g der Titelverbindung als farbloses Öl.
IR: 2941, 2875, 1450, 971, 948/cm

12 c) (1R,5S,6S,7R)-7-(Tetrahydropyran-2-yloxy)-6-[3S)-4,4-dimethyl-3-(tetrahydropyran-2-yloxy)-nona-1,6-diinyl]-bicyclo [3.3.0]octan-3-on

In Analogie zu Beispiel 8 c erhält man aus 3,30 g der nach Beispiel 12 b hergestellten Substanz 2,15 g der Titelverbindung als farbloses Öl.
IR: 2950, 2872, 2210, 1737, 1022, 970, 905, 870/cm

12 d) 2-{(Z)-(1S,5S,6S,7R)-7-(tetrahydropyran-2-yloxy)-6-[(3S)-4,4-dimethyl-3-(tetrahydropyran-2-yloxy)-nona-1,6-diinyl]-bicyclo[3.3.0]octan-3-yliden)}2-fluor-äthan-1-ol

In Analogie zu Beispiel 8 d erhält man aus 2 g des nach Beispiel 12 c hergestellten Ketons nach chromatographischer Isomerentrennung 435 mg unpolareres 2-{(E)-(1S,5S,6S,7R)-7-(Tetrahydropyran-2-yloxy)-6-[(3S)-4,4-dimethyl-3-(tetrahydropyran-2-yloxy)-nona-1,6-diinyl]-bicyclo[3.3.0]octan-3-yliden}-2-fluor-äthan-1-ol und als polarere Komponente 570 mg der Titelverbindung als farbloses Öl.
IR: 3600, 3410, 2938, 2217, 1138, 1018, 970, 871/cm

**Beispiel 13**

**(5Z)-(16RS)-5-Fluor-16-methyl-3-oxa-18,18,19,19-tetradehydro-6a-carba-prostaglandin-$I_2$-methylester**

100 mg (5Z)-(16RS)-5-Fluor-16-methyl-3-oxa-18,18,19,19-tetradehydro-6a-carba-prostaglandin-$I_2$ löst man in, 20 ml Dichlormethan und tropft bei 0°C bis zur bleibenden Gelbfärbung eine ätherische Diazomethanlösung zu. Nach 5 Minuten wird im Vakuum eingedampft und der Rückstand an Kieselgel adsorbiert. Man eluiert mit Hexan/ Ethylacetat 20-70 % und erhält 82 mg der Titelverbindung als farbloses Öl.
IR: 3600, 3410, 2955, 1741, 976/cm

**Beispiel 14**

**(5Z)-(16RS)-5-Fluor-16-methyl-3-oxa-18,18,19,19-tetradehydro-6a-carba-prostaglandin-$I_2$-carboxamid**

Eine Lösung von 185 mg (5Z)-(16RS)-5-Fluor-16-methyl-3-oxa-18,18,19,19-tetradehydro-6a-carba-prostaglandin-$I_2$ (siehe Beispiel 1) in 5 ml Dimethylformamid wird bei 0°C mit 60 mg Triäthylamin und 80 mg Chlorameisensäureisobutylester versetzt. Nach 30 Minuten wird 15 Minuten trockenes Ammoniak-Gas eingeleitet. Man läßt 2 Stunden bei 20°C stehen, verdünnt mit Citratpuffer (pH5), extrahiert mehrere Male mit Essigester, wäscht den Extrakt mit Natriumhydrogencarbonatlösung und Sole, trocknet über Magnesiumsulfat und dampft im Vakuum ein. Nach Chromatographie des Rückstandes an Kieselgel mit Dichlormethan/1-5 % Isopropanol und erhält 135 mg der Titelverbindung als farbloses Öl.
IR: 3600, 3510, 3410, 2953, 1668, 976/cm

**Beispiel 15**

**(5Z)-(16RS)-5-Fluor-16-methyl-3-oxa-18,18,19,19-tetradehydro-6a-carba-prostaglandin-$I_2$-(2,3-dihydroxy-propyl)-amid**

345 mg (5Z)-(16RS)-5-Fluor-16-methyl-3-oxa-18,18,19,19-tetradehydro-6a-carba-prostaglandin-$I_2$-11,15-bis-(tetrahydropyranyläther) werden in 5 ml Aceton gelöst und bei 0°C mit 74 mg Triäthylamin und 100 mg Chlorameisensäureisobutylester versetzt. Nach 20 Minuten fügt man eine Lösung von 574 mg 1-Amino-2,3-dihydroxypropan in 10 ml Aceton und 10 ml Acetonitril zu und rührt 1 Stunde bei 20°C. Man engt im Vakuum ein, verdünnt mit 100 ml Dichlormethan, wäscht mit Sole, trocknet über Magnesiumsulfat und dampft im Vakuum ein.
Den Rückstand rührt man 18 Stunden bei 20°C mit 10 ml Essigsäure/ Wasser-Tetrahydrofuran (65/35/10), dampft im Vakuum ein und reinigt den Rückstand durch Säulenchromatographie an Kieselgel. Mit Dichlormethan/Isopropanol (5-20 %) eluiert man 253 mg der Titelverbindung als farbloses Öl.
IR: 3600, 3390, 2931, 1642, 976/cm

**Beispiel 16**

**(5Z)-(16RS)-5-Fluor-16-methyl-3-oxa-18,18,19,19-tetradehydro-6a-carba-prostaglandin-$I_2$-acetylamid**

Zu einer Lösung von 265 mg (5Z)-(16RS)-5-Fluor-16-methyl-3-oxa-18,18,19,19-tetradehydro-6a-carba-prostaglandin-$I_2$-11,15-bis-(tetrahydropyranyläther) (siehe Beispiel 1) in 8 ml Acetonitril gibt man bei 20°C 65 mg Triäthylamin, kühlt auf 0°C ud tropft eine Lösung von 48 mg Acetylisocyanat in 5 ml Acetonitril zu. Nach 2 Stunden bei 20°C engt man im Vakuum ein, verdünnt mit 100 ml Citratpuffer (pH5), extrahiert mit Äther, wäscht den Extrakt nacheinander mit Natriumhydrogencarbonatlösung und Sole, trocknet über Magnesiumsulfat und dampft im Vakuum ein. Zur Abspaltung der Schutzgruppen rührt man den Rückstand mit 10 ml Eisessig/Wasser/Tetrahydrofuran (65/35/10) über Nacht und dampft im Vakuum zur Trockne. Der Rückstand wird an Kieselgel mit Dichlormethan/1 % Isöpropanol chromatographiert. Man erhält 105 mg der Titelverbindung als farbloses Öl.

IR: 3600, 3405, 1707, 972/cm

**Beispiel 17**

**(5Z)-(16RS)-5-Fluor-16-methyl-3-oxa-18,18,19,19-tetradehydro-6a-carba-prostaglandin-$I_2$-(4-phenyl)-phenacylester**

15 mg (5Z)-(16RS)-5-Fluor-16-methyl-3-oxa-18,18,19,19-tetradehydro-6a-carba-prostaglandin-$I_2$ werden in 3 ml Aceton gelöst und mit 87 mg ω-Brom-4-phenylacetophenon und 1 ml Triäthylamin versetzt und über Nacht bei Raumtemperatur unter Argon gerührt. Man versetzt mit 200 ml Äther, schüttelt zweimal mit je 10 ml Wasser, trocknet über Magnesiumsulfat und dampft im Vakuum ein. Die Reinigung erfolgt durch präparative Schichtchromatographie an Kieselgelplatten, die mit Dichlormethan/10 % Isopropanol entwickelt werden. Man erhält 152 mg der Titelverbindung als Öl.

IR: 3600, 2938, 1740, 1702, 1601, 974/cm

**Beispiel 18**

**(5Z)-(16RS)-5-Fluor-16-methyl-3-oxa-18,18,19,19-tetradehydro-6a-carba-prostaglandin-$I_2$-tris-(hydroxymethyl)-aminomethan-salz**

Zu einer Lösung von 208 mg (5Z)-(16RS)-5-Fluor-16-methyl-3-oxa-18,18,19,19-tetradehydro-6a-carba-prostaglandin-$I_2$ in 35 ml Acetonitril gibt man bei 68°C eine Lösung von 60 mg Tris-(hydroxymethyl)-aminomethan in 0,2 ml Wasser. Man läßt unter Rühren abkühlen, dekantiert nach 16 Stunden vom Lösungsmittel und trocknet den Rückstand im Vakuum. Man isoliert 192 mg der Titelverbindung als zähes Öl.

**Patentansprüche**

1. Carbacyclinderivate der allgemeinen Formel I

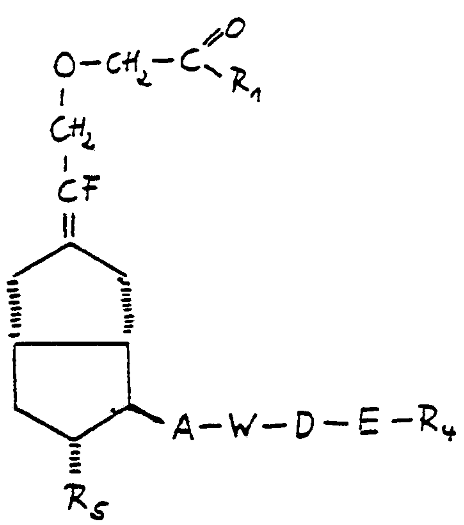

(I),

$R_1$ den Rest $OR_2$, wobei $R_2$ Wasserstoff, Alkyl mit 1-4 C-Atomen, Cycloalkyl mit 5-6 C-Atomen, Phenyl, 1-Naphthyl, 2-Naphthyl, den Rest

$$-CH_2-\overset{\overset{O}{\|}}{C}-Aryl,$$

wobei Aryl einen durch Phenyl, $C_1$-$C_2$-Alkoxy, Chlor oder Brom substituierten Phenyl-, 1-Naphthyl- oder 2-Napthylrest darstellt, oder $R_1$ eine 5- oder 6-gliedrige heterocyclische Gruppe mit einem N-, O- oder S-Atom bedeuten kann, oder $R_1$ den Rest $NHR_3$ mit $R_3$ in der Bedeutung eines organischen Carbon- oder Sulfonsäurerestes mit 1-15 C-Atomen oder des Restes $R_2$,

A eine $-CH_2-CH_2-$, trans-$CH=CH-$ oder $-C\equiv C-$Gruppe,

W eine freie oder funktionell abgewandelts Hydroxymethylengruppe oder eine freie oder funktionell abgewandelte

$$-\overset{CH_3}{\underset{OH}{C}}- \text{ Gruppe},$$

wobei die OH-Gruppe α- oder β-ständig sein kann,

D die Gruppe

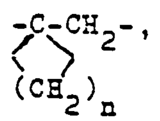

eine geradkettige gesättigte Alkylengruppe mit 1-5 C-Atomen, eine verzweigte gesättigte oder eine geradkettige oder verzweigts ungesättigte Alkylengruppe mit 2-5 C-Atomen, die gegebenenfalls durch Fluoratome substituiert sein können,

n die Zahl 1, 2 oder 5 bedeuten kann,

E eine Direktbindung, eine $-C\equiv C-$Gruppe oder eine $-CR_6=CR_7-$Gruppe darstellt, wobei $R_6$ Wasserstoff oder $C_1$-$C_4$-Alkyl und $R_7$ Wasserstoff, $C_1$-$C_4$-Alkyl Chlor oder Brom bedeuten,

$R_4$ eine Alkylgruppe mit 1-10 C-Atomen, eine Cycloalkylgruppe mit 3-6 C-Atomen, Phenyl oder eine 5- oder 6-gliedrige heterocyclische Gruppe mit einem N-, O- oder S-Atom,

$R_5$ eine freie oder funktionell abgewandelte Hydroxygruppe, und

falls $R_2$ die Bedeutung eines Wasserstoffatoms hat, deren Salze mit physiologisch verträglichen Basen bedeuten.

2. Verfahren zur Herstellung der Carbacyclinderivate der allgemeinen Formel I, dadurch gekennzeichnet, daß man in an sich bekannter Weise eine Verbindung der allgemeinen Formel II,

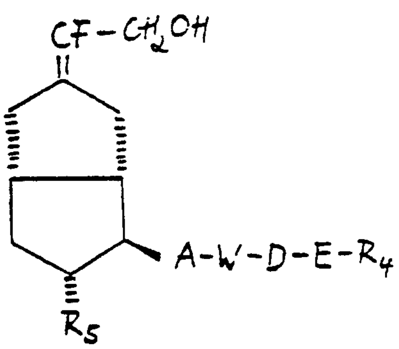

II,

worin $R_4$, $R_5$, A, W, D und E die obenangegebenen Bedeutungen aufweisen, gegebenenfalls nach Schutz anwesender freier Hydroxygruppen mit eine, Halogenessigsäurederivat der allgemeinen Formel III,

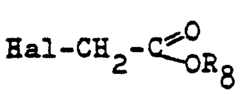

III,

wobei Hal ein Chlor- Brom- oder Jodatom und $R_8$ einen Alkylrest oder Trialkyl-silylrest mit $C_1$-$C_4$-Alkylgruppen oder ein Alkalimetall (Na, Li, K) bedeutet, in Gegenwart einer Base veräthert und gegebenenfalls anschließend in beliebiger Reihenfolge Isomere trennt und/oder geschützte Hydroxygruppen freisetzt und/oder freie

Hydroxygruppen verestert oder veräthert und/oder eine freie Carboxylgruppe verestert und/oder eine veresterte Carboxylgruppe verseift oder eine Carboxylgruppe in ein Amid oder mit einer physiologisch verträglichen Base in ein Salz überführt.

3. Arzneimittel, bestehend aus einer oder mehreren Verbindungen des Anspruches 1 und üblichen Hilfs- und Trägerstoffen.

4. (5Z)-(16RS)-5-Fluor-16-methyl-3-oxa-18,18,19,19-tetradehydro-6a-carba-prostaglandin-$I_2$

5. (5Z)-(16RS)-13,14-Didehydro-16,20-dimethyl-c-fluor-3-oxa-18,18,19,19-tetradehydro-6a-carba-prostaglandin-$I_2$

## Claims

1. Carbacyclin derivatives of the general formula I:

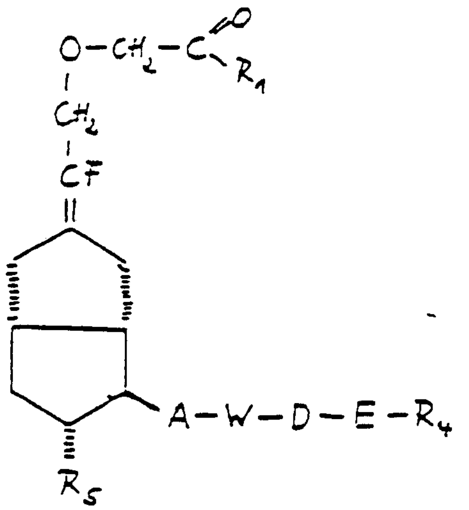

(I),

wherein

$R_1$ is the group $OR_2$, in which $R_2$ is hydrogen alkyl of 1-4 C-atoms, cycloalkyl of 5-6 C-atoms, phenyl, 1-naphthyl or 2-naphthyl, the group

$$-CH_2-\overset{\overset{\displaystyle O}{\|}}{C}-aryl,$$

in which aryl is phenyl, 1-naphthyl or 2-naphthyl, each of which is substituted by phenyl, $C_1$-$C_2$-alkoxy, chloro or bromo, or $R_1$ is a 5 or 6 membered heterocyclic group with an N-, O- or S-atom, or $R_1$ is the group $NHR_3$, in which $R_3$ is an organic, carboxylic or sulphonic acid ester group or is the group $R_2$,

A is a $-CH_2-CH_2-$, trans $-CH{=}CH-$ or $-C{\equiv}C-$group,

W is a free or functionally modified hydroxymethylene group or a free or functionally modified

$$\begin{matrix} CH_3 \\ -C- \\ OH \end{matrix} \text{ group,}$$

where the OH group may be in the α- or β-configuration,

D is the group

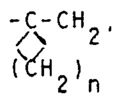

a straight-chain saturated alkylene group of 1-5 C-atoms or a branched, saturated or a straight-chain or branched unsaturated alkylen, group of 2-5 C-atoms, which can optionally be substituted by fluorine atoms,

n is the number 1, 2 or 3,

E is a direct bond, a $-C{\equiv}C-$ group or a $-CR_6{=}CR_7-$ group, where $R_6$ is hydrogen or a $C_{1\text{-}4}$-alkyl group and $R_7$ is hydrogen, $C_{1\text{-}4}$-alkyl, chlorine or bromine,

$R_4$ is an alkyl group of 1-10 C-atoms, a cycloalkyl group of 3-6 C-atoms, phenyl or 5 or 6 membered heterocyclic group with an N-, O- or S-atom,

$R_5$ is a free or functionally modified hydroxy group, and when $R_2$ is hydrogen, its salts with physiologically acceptable bases.

2. Process for the preparation of carbacyclin derivatives of general formula I, characterised in that, in known manner, a compound of the general formula II

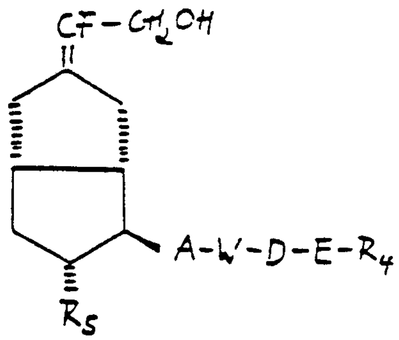

II

wherein $R_4$, $R_5$, A, W, D and E have the meanings given above, is etherified, in the presence of a base optionally after protection of hydroxy groups which are present with a haloacetic acid derivative of general formula III,

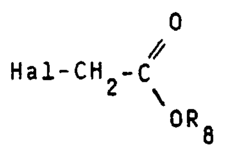

III

in which Hal is a chlorine, bromin, or iodin, atom and $R_8$ is an alkyl or trialkylsilyl group with $C_1$-$C_4$-alkyl groups or an alkali metal (Na, Li, K) and optionally, subsequently and in any preferred order, isomers are separated and/or protected hydroxy groups are freed and/or free hydroxy groups are esterified or etherified and/or a free carboxyl group is esterified and/or an esterified carboxyl group is saponified or a carboxyl group is converted to an amide or, with a physiologically acceptable base, to a salt.

3. Pharmaceuticals containing one or more compounds according to claim 1 and conventional additives and carriers.

4. (5Z)-(16RS)-5-Fluoro-16-methyl-3-oxa-18,18,19,19-tetradehydro-6a-carbaprostaglandin-$I_2$

5. (5Z)-(16RS)-13,14-Didehydro-16,20-dimethyl-5-fluoro-3-oxa-18,18,19,19-tetradehydro-6a-carbaprostaglandin-$I_2$

**Revendications**

1. Carbacyclines de formule générale I ci-dessous

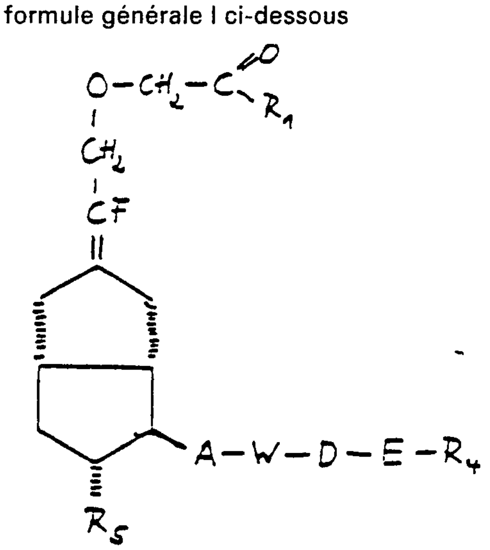

(I),

dans laquelle:

$R_1$ représente un radical $OR_2$, $R_2$ étant l'hydrogène, un alkyle en $C_1$ à $C_4$, un cycloalkyle en $C_5$ ou $C_6$ ou un groupe phényle, 1-naphtyle, 2-naphtyle ou

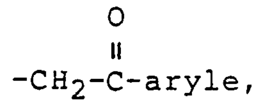

l'aryle étant un groupe phényle, 1-naphtyle ou 2-naphtyle substitué par un phényle, un alcoxy en $C_1$ ou $C_2$ ou un atome de chlore ou de brome, ou encore un groupe hétérocyclique pentagonal ou hexagonal avec un atome d'azote, d'oxygène ou de soufre, ou bien $R_1$ représente un radical $NHR_3$, $R_3$ étant un groupe d'acide carboxylique ou sulfonique organique en $C_1$ à $C_{15}$ ou le radical $R_2$,

A désigne un groupe $-CH_2-CH_2-$, trans-$CH\equiv CH$- ou $-C\equiv C-$,

W un groupe hydroxyméthylène libre ou à fonction modifiée ou un groupe

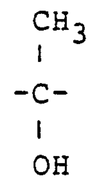

libre ou à fonction modifiée, le groupe OH pouvant être à la position α ou β,

D le groupe

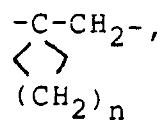

un alkylène saturé à chaîne linéaire en $C_1$ à $C_5$ ou un alkylène en $C_2$ à $C_5$ saturé et ramifié ou bien insaturé et linéaire ou ramifié, pouvant être éventuellement substitués par des atomes de fluor,

n pouvant être le nombre 1, 2 ou 3,

E désigne une liaison directe, un groupe $-C\equiv C-$ ou un groupe $-CR_6=CR_7-$, $R_6$ étant l'hydrogène ou un alkyle en $C_1$-$C_4$ et $R_7$ l'hydrogène, un alkyle en $C_1$-$C_4$ ou un atome de chlore ou de brome,

$R_4$ un alkyle en $C_1$-$C_{10}$, un cycloalkyle en $C_3$-$C_6$, un phényle ou un groupe hétérocyclique pentagonal ou hexagonal avec un atome d'azote, d'oxygène ou de soufre, et

$R_5$ un hydroxyle libre ou à fonction modifiée),

ainsi que, dans le cas où $R_2$ est un atome d'hydrogène, les sels de ces carbacyclines formés avec des bases physiologiquement compatibles.

2. Procédé de préparation des carbacyclines de formule I selon la revendication 1, procédé caractérisé en ce que l'on éthérifie de manière connue un composé de formule générale II :

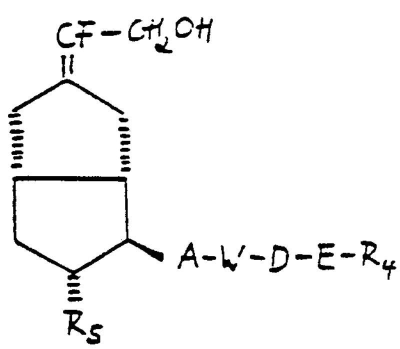

II

éventuellement après en avoir protégé les hydroxyles libres présents, avec un dérivé d'acide halogénoacétique de formule III:

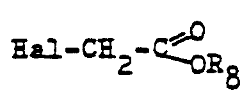

III,

(Hal désignant un atome de chlore, de brome ou d'iode et $R_8$ un radical alkyle ou trialkylsilyle en $C_1$ à $C_4$ dans le groupe alkyle ou encore un métal alcalin tel que Na, Li ou K) en présence d'une base, puis le cas échéant, et en procédant dans un ordre quelconque, on sépare les isomères et/ou on libère les hydroxyles protégés et/ou on estérifie ou étherifie des hydroxyles libres et/ou on estérifie un groupe carboxylique libre et/ou encore on saponifie un groupe carboxylique estérifié ou on transforme un groupe carboxylique en un groupe amide ou on le salifie avec une base compatible du point de vue physiologique.

3. Médicament comprenant un ou plusieurs composés de la revendication 1 avec les excipients et véhicules usuels et pharmacie galénique.

4. (5Z)-(16RS)-5-Fluoro-16-méthyl-3-oxa-18,18,19,19-tétradéhydro-6a-carba-prostaglandine-$I_2$ selon la revendication 1.

5. (5Z)-(16RS)-13,14-Didéhydro-16,20-diméthyl-5-fluoro-3-oxa-18,18,19,19-tétradéhydro-6a-carba-prostaglandine-$I_2$ selon la revendication 1.